(19) Europäisches Patentamt
European Patent Office
Office européen des brevets

(11) Veröffentlichungsnummer : **0 003 825**
**B1**

(12) # EUROPÄISCHE PATENTSCHRIFT

(45) Veröffentlichungstag der Patentschrift : **11.02.81**

(51) Int. Cl.³ : **C 07 C 59/52**, C 07 C 51/00, C 07 C 59/56

(21) Anmeldenummer : **79100497.1**

(22) Anmeldetag : **20.02.79**

(54) **Verfahren zur Herstellung von 4-Hydroxyphenylessigsäure.**

(30) Priorität : **20.02.78 DE 2807201**

(43) Veröffentlichungstag der Anmeldung : **05.09.79 (Patentblatt 79/18)**

(45) Bekanntmachung des Hinweises auf die Patenterteilung : **11.02.81 Patentblatt 81/06**

(84) Benannte Vertragsstaaten : **BE CH DE FR GB IT NL**

(56) Entgegenhaltungen :
**AUSTRALIAN JOURNAL OF CHEMISTRY, Vol. 26, Nr. 10, Oktober 1973, East Melbourne, L. BREEN et al. : « Formation of arylacetic acids, arylacetamides and aryldihydropyrroles from aromatic aldehydes », Seiten 2221-2227**

(73) Patentinhaber : **Diamalt Aktiengesellschaft
Friedrichstrasse 18
D-8000 München (DE)**

(72) Erfinder : **Kaniss, Normann, Dr.
Petersbergweg 1
D-8201 Raubling (DE)**
Erfinder : **Bauer, Adolf, Dr.
Wendelsteinstrasse 6c
D-8201 Raubling (DE)**

(74) Vertreter : **Lederer, Franz, Dr. et al
Patentanwälte Dr. Lederer Franz Meyer-Roxlau Reiner F. Lucile-Grahn-Strasse 22
D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Verfahren zur Herstellung von 4-Hydroxyphenylessigsäure

4-Hydroxyphenylessigsäure ist ein wichtiges Zwischenprodukt für die Synthese pharmazeutischer Wirkstoffe. Es besteht daher ein allgemeines Interesse an einer möglichst rationellen Synthese.

Bekannte Verfahren gehen beispielsweise von Anisol aus, das durch Chlormethylierung und Cyanidierung in 4-Methoxyphenylacetonitril übergeführt wird (zit. nach Organikum, Berlin, 9. Auflage, S. 363). Die Verseifung des Nitrils zur Säure und deren anschließende Etherspaltung mit Phosphor/Jodwasserstoffsäure (R. J. MELTZER et al., J. Org. Chem. *22* (1957) 1577) führt zur 4-Hydroxyphenylessigsäure. Die 4-stufige Synthese verläuft jedoch mit sehr unbefriedigenden Ausbeuten. Die Gesamtausbeute, auf Anisol bezogen, liegt im allgemeinen unterhalb von 21 %.

Ein anderer bekannter Weg führt, von Phenol ausgehend, durch Friedel-Crafts-Reaktion zu 4-Hydroxyacetophenon, das nach Willgerodt-Kindler in die Zielverbindung übergeführt wird (C. D. JOSHI et al., J. Sci. Ind. Res. (New Delhi) Sect. B *21* (1962 284). Auch hier ist die Gesamtausbeute, auf Phenol bezogen, mit ca. 30 % äußerst unbefriedigend. Zudem tritt mit den als Nebenprodukten anfallenden Sulfiden ein Abwasserproblem auf, dessen Beseitigung einen unverhältnismäßig hohen Aufwand erfordert.

Es war daher die Aufgabe gestellt, ein Verfahren zu finden, das ohne die genannten Nachteile und in guten Ausbeuten zu 4-Hydroxyphenylessigsäure führt. Dabei stellte sich überraschenderweise heraus, daß ein zunächst als umständlicher Umweg erscheinendes Verfahren diese Aufgabe zufriedenstellend löst.

Das erfindungsgemäße Verfahren ist dadurch gekennzeichnet, daß man

a) o-Chlorphenol mit Glyoxylsäure zu 3-Chlor-4-hydroxy-mandelsäure umsetzt,
b) die 3-Chlor-4-hydroxy-mandelsäure zu 3-Chlor-4-hydroxy-phenyl-essigsäure reduziert und
c) aus der 3-Chlor-4-hydroxy-phenylessigsäure das Chlor abspaltet.

Die Umsetzung von Phenol mit Glyoxylsäure ist beispielsweise aus Houben-Weyl, Methoden der organischen Chemie, Band VI/1 c (1976), Seiten 1057-1058 und Canad. J. Chem. 44, 1966, Seiten 575-582 bekannt. Die hierbei entstehende 4-Hydroxy-mandelsäure läßt sich jedoch nicht auf einfache Weise zur 4-Hydroxy-phenyl-essigsäure reduzieren.

Die Anmelderin hat nun gefunden, daß sich 3-Chlor-4-hydroxymandelsäure ebenfalls nach diesem für die Herstellung von 4-Hydroxy-mandelsäure bekannten Verfahren herstellen läßt, wobei jedoch im Gegensatz zu der nicht-chlorierten Verbindung sich die 3-Chlor-4-hydroxy-mandelsäure glatt zu 3-Chlor-4-hydroxy-phenylessigsäure reduzieren läßt, aus der das Chlor leicht reduzierend abgespalten werden kann.

Aus der BE-PS 704 368 ist es bekannt, mono- oder disubstituierte Phenole entweder nach Friedel-Crafts zum entsprechenden Acetophenonderivat und dann nach Willgerodt-Kindler über das Thiomorpholid-Derivat zur entsprechenden Phenylessigsäure umzusetzen oder aber nach Einführung geeigneter Schutzgruppen durch Chlormethylierung und Cyanidierung und anschließende Verseifung in Phenylessigsäuren überzuführen. Ein solches Verfahren wurde gemäß DE-OS 25 24 836 dadurch verbessert, daß das o-Chlorphenol zum Schutz der Hydroxygruppe mit einem sekundären Alkylhalogenid in 2-Chloralkoxybenzol umgewandelt wird, welches dann chlormethyliert, cyanidiert und weiterverarbeitet wird. Obwohl dieses Verfahren in Durchführung und Ausbeute den früheren Verfahren überlegen ist, konnte es auch nicht voll befriedigen. Einmal benötigen diese bekannten Verfahren schon bis zur Stufe der 3-Chlor-4-hydroxyphenyl-essigsäure mindestens vier bzw. fünf Stufen, erfordern dadurch entsprechend großen Arbeitsaufwand und erbringen auch nur mäßige Ausbeuten von weniger als 50 %, und zum anderen durchlaufen sie für Umwelt und Gesundheit so risikoreiche Verfahrensstufen wie die Bildung des Thiomorpholids bzw. die Cyanidierung der Chlormethylstufe. Die Zersetzungs-produkte der Thiomorpholid-Derivate belasten voll das Abwasser und stellen für die biologische Klärung ein besonderes Problem dar. Die Mutterlaugen des Cyanidierungsprozesses, der an sich schon besondere Vorsichtsmaßnahmen erfordert, bedürfen einer speziellen Behandlung zur praktisch quantitativen Beseitigung des überschüssigen Cyanids. Setzt man o-Chlorphenol nach dem Verfahren der DE-OS 25 24 836, zu 3-Chlor-4-hydroxyphenyl-essigsäure um, so kann man daraus durch reduktive Chlorabspaltung die gewünschte 4-Hydroxyphenyl-essigsäure in einer auf o-Chlorphenol bezogenen Ausbeute von nur ca. 40 % erhalten, während der naheliegende Syntheseweg, der analog zur Herstellung von 3-Chlor-4-hydroxyphenyl-essigsäure von Phenol direkt ausgehend über die entsprechenden Zwischenstufen verläuft, zu gar keinen befriedigenden Ergebnissen führt.

Die Umsetzung von o-Chlorphenol mit Glyoxylsäure in Stufe a) des erfindungsgemäßen Verfahrens wird in alkalischer Lösung vorgenommen. Der Wahl der Mol-Verhältnisse sind keine Grenzen gesetzt, vorzugsweise verwendet man einen Überschur der Glyoxylsäure. Die Reaktion läuft bei Temperaturen zwischen − 5 und 100 °C ab. Nach beendeter Reaktion setzt man die Mandelsäure durch Ansäuern in Freiheit, extrahiert gegebenenfalls mit einem geeigneten Extraktionsmittel oder setzt das Reaktionsprodukt direkt weiter um.

Nachdem bekannt war, daß zahlreiche andere substituierte Phenole, wie z.B. Alkoxyphenole mit Glyoxylsäure und analogen Verbindungen harzartige Produkte oder völlig unzureichende Ausbeuten liefern, war es überraschend, daß

Glyoxylsäure das o-Chlorphenol ausschließlich in para-Stellung zur phenolischen Hydroxygruppe angreift und ausgezeichnete Ausbeuten gewährleistet. Dies ist insbesondere überraschend, weil bekannt ist, daß Glyoxylsäure und ähnliche Verbindungen in alkalischer Lösung disproportionieren, daß ungeschützte Phenole zu Autoxidation und Verharzung neigen, insbesondere im Gemisch mit Aldehyden, und eine Umsetzung von Chlorphenol mit einer Verbindung wie Glyoxylsäure Isomerenbildung und schwierige Trennverfahren erwarten läßt. Die geringe Kristallisationsfreudigkeit der phenolischen Mandelsäuren stellt darüber hinaus die Weiterverarbeitung der Zwischenprodukte infrage.

Für die Reduktion der 3-Chlor-4-hydroxy-mandelsäure zu 3-Chlor-4-hydroxy-phenylessigsäure in Stufe b) des erfindungsgemäßen Verfahrens eignen sich verschiedene Reduktionsmittel, die von der Reduktion von α-Halogenphenylessigsäuren und Mandelsäuren zu den entsprechenden Phenylessigsäuren her bekannt sind, so die Hydrierung an Edelmetallkatalysatoren, die Reduktion mit Natriumborhydrid, die Reduktion mit Wasserstoff in statu nascendi, der Austausch der OH-Gruppe durch einen Halogensubstituenten, der mit Zinn/Salzsäure oder Zink/Eisessig oder mit Wasserstoff/Katalysator entfernt werden kann oder die Reduktion mit Jodwasserstoffsäure bzw. Jod und Phosphor. Die bevorzugte Reduktion mit Jodwasserstoffsäure geht in einem geeigneten Lösungsmittel wie Eisessig vor sich, wobei die Jodwasserstoffsäure auch ganz oder teilweise durch Jod + Phosphor ersetzt werden kann. Zweckmäßigerweise stellt man das die 3-Chlor-4-hydroxyphenylessigsäure enthaltende Reaktionsmedium mit Natronlauge alkalisch, läßt das Mononatriumsalz auskristallisieren, welches gewaschen und in wenig heißem Wasser wieder aufgelöst wird, worauf nach Ansäuern und Abkühlen die 3-Chlor-4-hydroxyphenylessigsäure kristallin abgeschieden wird.

Die Abspaltung des Chlors in der Stufe c) des erfindungsgemäßen Verfahrens kann auf verschiedene Weise durchgeführt werden, vorzugsweise jedoch katalytisch mit Wasserstoff und einem Palladiumkatalysator.

Aus der Literatur ist eine ganze Reihe von Methoden und Beispielen bekannt, Halogen in organischen Verbindungen durch Wasserstoff zu substituieren.

Neben der Hydrierung am Palladiumkatalysator kann man auch an Raney-Nickel in alkalischer Lösung hydrierend Halogen abspalten, oder man kann mit Jodwasserstoffsäure und Phosphor oder auch mit Natrium in flüssigem Ammoniak arbeiten, auch die Reduktion mit naszierendem Wasserstoff ist bekannt. Die Dehalogenierung im aromatischen System erfordert meist drastische Bedingungen und/oder potente Reduktionsmittel, was jedoch dazu führen kann, daß u.U. Doppelbindungen hydriert werden können oder auch weitere funktionelle Gruppen neben dem Halogen angegriffen und reduktiv eliminiert werden können.

Die Dehalogenierung von 3-Chlorbenzoesäure zu Benzoesäure mit Wasserstoff/Raney-Nickel geht beispielsweise (Chem. Ber. *91* (1958) 1376) nur mit einer Ausbeute von 64 % gegenüber der Reduktion von Chloranilinen und Chloranisolen, die mit ca. 90 % Ausbeute verläuft.

Im Vergleich dazu ergibt die Reduktion der 3-Chlor-4-hydroxyphenylessigsäure in der Stufe c des erfindungsgemäßen Verfahrens nach der vorzugsweisen Methode das Zielprodukt in einer Ausbeute von über 90 %.

Man arbeitet in alkalischer Lösung und bei Temperaturen, die von Raumtemperatur bis zur Siedetemperatur des Reaktionsgemischs reichen, wobei der Wasserstoffdruck dem Außendruck entsprechen oder ihn übersteigen kann.

Die Gesamtausbeute des erfindungsgemäßen Verfahrens liegt bei etwa 65 %, bezogen auf eingesetztes o-Chlorphenol.

Das erfindungsgemäße Verfahren kann durch das nachstehende Reaktionsschema erläutert werden :

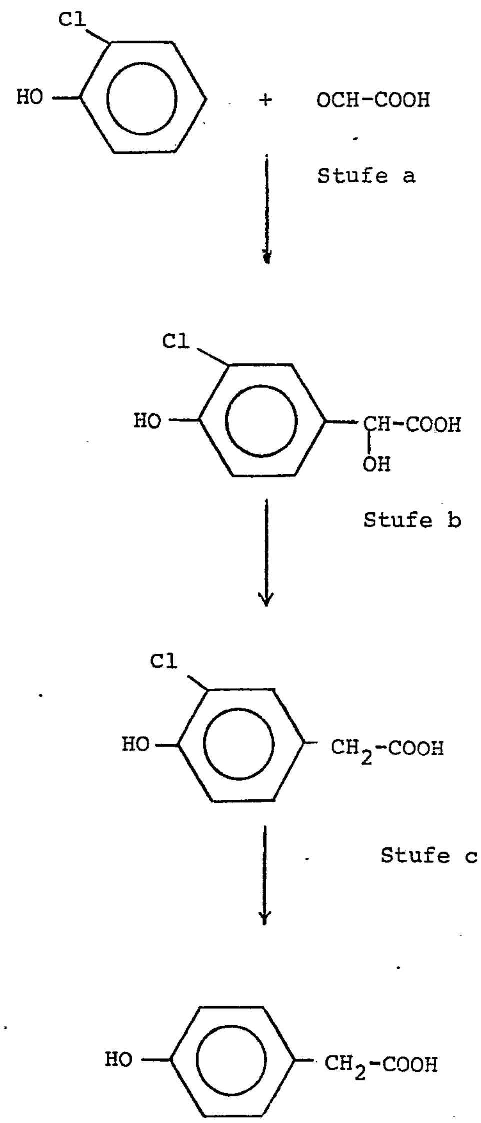

4-Hydroxyphenylessigsäure wird zur Herstellung verschiedener pharmazeutischer Wirkstoffe verwendet.

Gemäß US-PS 2 487 018 wird p-Hydroxybenzylpenicillin-Natrium durch Zugabe von 4-Hydroxyphenylessigsäure zu einer Penicilliumkultur erhalten. p-Hydroxybenzylpenicillin-Natrium ist antibiotisch wirksam.

Gemäß GB-PS 1 285 038 wird 4-Hydroxyphenylessigsäure zu 1-p-Carbamoylmethylphenoxy-2,3-epoxypropan umgesetzt, aus dem durch Reaktion mit Isopropylamin der Wirkstoff Atenolol entsteht, der als Beta-Blocker eingesetzt wird.

Gemäß DE-OS 26 21 090 werden ausgehend von 4-Hydroxyphenylessigsäure 2-(p-Hydroxyphenyl)-3-amino-1-propanol-derivate mit broncholytischer Aktivität erhalten.

Nach J. Med. Chem. *20,* 1263 ff (1977) substituiert man 4-Hydroxyphenylessigsäure zunächst in 3-Stellung, setzt nach Schutzgruppeneinführung mit tert.-Butyl-benzylamin um und reduziert das Reaktionsprodukt zu Verbindungen mit Betaadrenergica ähnlicher Wirkung.

Beispiel

In einem geeigneten Reaktionsgefäß legt man 7 l Wasser vor, gibt 1,75 kg 50 %-ige Glyoxylsäurelösung zu und neutralisiert mit 1,63 l 25 %-iger Natronlauge. Dazu gibt man bei Raumtemperatur eine Mischung von 0,39 kg o-Chlorphenol und 0,38 l 25 %-iger Natronlauge, erwärmt auf ca. 30 °C und läßt bei dieser Temperatur unter Rühren ca. 24 St. reagieren. Man stellt mit konz. Salzsäure den pH-Wert auf 1-2, dampft im Vakuum auf etwa die Hälfte des Volumens ein, kühlt und extrahiert dreimal mit je 1,5 l Essigester. Die vereinigten Essigesterextrakte werden im Vakuum weitgehend eingeengt. Der Rückstand bildet eine helle, harzartige Masse von 615 g 3-Chlor-4-hydroxymandelsäure (entspr. 98 % d.Th.), die bei Stehenlassen in einigen Tagen durchkristallisiert. Eine aus wenig Wasser umkristallisierte Probe der 3-Chlor-4-hydroxymandelsäure hat einen Schmelzpunkt von 140-141 °C.

Analyse : Cl gef. : 17,7 und 17,3 % Cl ber. : 17,53 %.

Die Substanz ist im Dünnschichtchromatogramm einheitlich. 500 g der rohen 3-chlor-4-hydroxymandelsäure werden in 2,5 l Eisessig gelöst, mit 87 g rotem Phosphor, 31 g Jod, 30 ml 57 %-iger Jodwasserstoffsäure und 30 ml Wasser versetzt und 2,5 Stunden unter Rückfluß gekocht. Man saugt vom nicht umgesetzten Phosphor ab und engt im Vakuum weitgehend ein. Der Rückstand wird in 650 ml Wasser aufgenommen, mit 1 l 25 %-iger Natronlauge versetzt und auf pH 8-8,5 gestellt. Dabei kristallisiert das Natriumsalz der 3-Chlor-4-hydroxyphenylessigsäure aus. Dieses wird zur 3-Chlor-4-hydroxyphenylessigsäure aufgearbeitet, wobei man 320 g dieser Säure erhält. Die Substanz ist sehr rein und hat einen Schmelzpunkt von 109-110 °C.

Die Ausbeute über beide Stufen beträgt 68,3 % d.Th., bezogen auf das eingesetzte o-Chlorphenol.

180 g 3-Chlor-4-hydroxyphenylessigsäure werden in 350 ml Wasser suspendiert und mit 240 ml 25 %-iger Natronlauge versetzt. Man erwärmt, gibt 4 g Palladiumkohle dazu, verschließt das Reaktionsgefäß, spühlt mehrmals mit Stickstoff und gibt Wasserstoff auf. Gleichzeitg wird das Reaktionsgemisch erwärmt. Bei einem Druck von ca. 5 bar und einer Temperatur von etwa 100 °C läuft die Reaktion innerhalb von 7-8 Stunden ab. Man filtriert von der Kohle ab, stellt das Filtrat mit ca. 200 ml konz. Salzsäure auf pH 1-2 und rührt unter Kühlen einige Zeit nach und saugt die auskristallisierte Substanz ab. Man erhält nach Trocknung 162 g rohes Produkt. Nach Umkristallisation werden 129 g reine 4-Hydroxyphenylessigsäure (entspr. 88 % d.Th.) erhalten. Durch Aufarbeitung der Mutterlaugen werden weitere 10 g der reinen Säure gewonnen, so daß die Gesamtausbeute dieser Stufe 96 % d.Th. beträgt.

Die 4-Hydroxyphenylessigsäure weist einen Schmelzbereich von 148-152 °C auf und ist mit authentischer Substanz identisch.

**Ansprüche**

1. Verfahren zur Herstellung von 4-Hydroxyphenylessigsäure, dadurch gekennzeichnet, daß man

a) o-Chlorphenol mit Glyoxylsäure zu 3-Chlor-4-hydroxymandelsäure umsetzt,

b) die 3-Chlor-4-hydroxy-mandelsäure zu 3-Chlor-4-hydroxyphenylessigsäure reduziert und

c) aus der 3-Chlor-4-hydroxy-phenylessigsäure das Chlor abspaltet.

2. Verfahren nach Anspruch 1, dadurch gekennzeichnet, daß man die Umsetzung in Stufe a) in alkalischem Medium durchführt.

3. Verfahren nach einem der vorgehenden Ansprüche, dadurch gekennzeichnet, daß man die Reduktion der Stufe b) mit rotem Phosphor und Jod durchführt.

4. Verfahren nach einem der vorgehenden Ansprüche, dadurch gekennzeichnet, daß man die Chlorabspaltung in Stufe c) katalytisch mit Wasserstoff und einem Palladium-katalysator durchführt.

**Claims**

1. Process for the preparation of 4-hydroxyphenylacetic acid, characterized in that

a) o-chlorophenol is reacted with glyoxylic acid to yield 3-chloro-4-hydroxy-mandelic acid,

b) the 3-chloro-4-hydroxy-mandelic acid is reduced to yield 3-chloro-4-hydroxy-phenylacetic acid and

c) the chlorine is split off from the 3-chloro-4-hydroxyphenyl-acetic acid.

2. Process according to claim 1 characterized in that the reaction in step a) is performed in alkaline medium.

3. Process according to one of the preceding claims, characterized in that the reduction in step b) is performed with red phosphorus and iodine.

4. Process according to one of the preceding claims characterized in that the splitting-off of chlorine in step c) is performed catalytically with hydrogen and a palladium catalyst.

**Revendications**

1. Procédé pour la préparation d'acide 4-hydroxyphénylacétique, caractérisé en ce

a) qu'on fait réagir l'o-chlorophénol avec l'acide glyoxylique pour obtenir l'acide 3-chloro-4-hydroxymandélique,

b) on réduit l'acide 3-chloro-4-hydroxymandélique en acide 3-chloro-4-hydroxyphénylacétique et

c) on élimine le chlore de l'acide 3-chloro-4-hydroxyphénylacétique.

2. Procédé selon la revendication 1, caractérisé en ce qu'on mène la réaction de la phase a) dans un milieu alcalin.

3. Procédé selon la revendication 1 ou 2, caractérisé en ce qu'on effectue la réduction de la phase b) avec du phosphore rouge et de l'iode.

4. Procédé selon l'une quelconque des revendications 1 à 3, caractérisé en ce qu'on effectue l'élimination du chlore dans la phase c) par voie catalytique avec de l'hydrogène et un catalyseur au palladium.